# EUROPEAN PATENT SPECIFICATION

(11) **EP 2 039 363 B1**
(45) Date of publication and mention of the grant of the patent: **02.01.2013**
(21) Application number: 07745338.9
(22) Date of filing: 15.06.2007
(51) Int. Cl.: A61K 31/785, A61K 45/00, A61P 13/12

(54) **AGENT FOR PREVENTION AND/OR TREATMENT OF GLOMERULOPATHY**
MITTEL ZUR PRÄVENTION UND/ODER BEHANDLUNG VON GLOMERULOPATHIE
AGENT DE PRÉVENTION ET/OU TRAITEMENT DE GLOMÉRULOPATHIE

(30) Priority: 16.06.2006 JP 2006166854
(43) Date of publication of application: 25.03.2009
(73) Proprietor: Mitsubishi Tanabe Pharma Corporation, Osaka 541-8505 (JP)
(72) Inventor: YAMAMORI, Masahiko, Tsu-shi, Mie 514-0065 (JP)
(74) Representative: Merkle, Gebhard
(86) International application number: PCT/JP2007/062083
(87) International publication number: WO 2007/145308

(56) References cited:
- EP-A- 1 413 309
- WO-A-2005/032563
- JP-A- 09 295 941
- JP-A- 2005 523 910
- NAGANO NOBUO ET AL: "Sevelamer hydrochloride, a phosphate binder, protects against deterioration of renal function in rats with progressive chronic renal insufficiency." NEPHROLOGY DIALYSIS TRANSPLANTATION, vol. 18, no. 10, October 2003 (2003-10), pages 2014-2023, XP002554968 ISSN: 0931-0509
- DIAMOND J R ET AL: "CHOLESTYRAMINE RESIN AMELIORATES CHRONIC AMINONUCLEOSIDE NEPHROSIS" AMERICAN JOURNAL OF CLINICAL NUTRITION, vol. 51, no. 4, 1990, pages 606-611, XP002554969 ISSN: 0002-9165
- MCDONALD E L ET AL: "Oedema due to subacute nephritis treated with ion-exchange resins" PROCEEDINGS OF THE ROYAL SOCIETY OF MEDICINE, ACADEMIC PRESS, LONDON, GB, vol. 46, no. 1, 1 January 1953 (1953-01-01), pages 46-48, XP009125636 ISSN: 0035-9157
- ROSENHEIM M L ET AL: "Treatment of nephrotic syndrome with cation-exchange resins and high-protein low-sodium diet" LANCET THE, LANCET LIMITED. LONDON, GB, vol. 271, no. 6938, 18 August 1956 (1956-08-18), pages 313-319, XP009125657 ISSN: 0140-6736
- NARAZAKI K. ET AL.: 'Colestymine Toyo nite Kessei Cholesterol-chi no Chomei na Kaizen o Mita Tonyobyosei Jinsho ni yoru Nephrose Shokogun no Nirei' THE JOURNAL OF THE JAPAN DIABETIC SOCIETY vol. 43, no. 3, 2000, page 257 + ABSTR. NO. 145, XP003020552
- AOYAGI K.: 'Sojo Shikyutai Kokasho' SHINDAN TO CHIRYO vol. 80, 1992, pages 521 - 522, XP003020553
- NIMURA H. ET AL.: 'Byotaitbetsu ni Mita Koshikessho Chiryoho no Jissai, Nijisei Koshikessho Jin Shikkan ni Miru Cholesterol Kessho' MEDICAL PRACTICE vol. 20, no. 1, 2003, pages 157 - 159, XP003020554
- HIRAYAMA K. ET AL.: 'Angiotensin Henkan Koso Sogaiyaku to Angiotensin II Juyotai Kikkoyaku no Jin Hogo Sayo' IGAKU NO AYUMI vol. 197, no. 11, 2001, pages 870 - 872, XP003020555
- LAM K S ET AL: "Cholesterol-lowering therapy may retard the progression of diabetic nephropathy.", DIABETOLOGIA MAY 1995 LNKD- PUBMED:7489845, vol. 38, no. 5, May 1995 (1995-05), pages 604-609, XP009143834, ISSN: 0012-186X
- MUSO ERI ET AL: "Low density lipoprotein apheresis therapy for steroid-resistant nephrotic syndrome", KIDNEY INTERNATIONAL, SUPPLEMENT,, vol. 56, no. Suppl.71, 1 July 1999 (1999-07-01), pages S122-S125, XP009143645, ISSN: 0098-6577
- DOMINGUEZ JESUS H ET AL: "Studies of renal injury III: Lipid-induced nephropathy in type II diabetes", KIDNEY INTERNATIONAL, NATURE PUBLISHING GROUP, LONDON, GB, vol. 57, no. 1, 1 January 2000 (2000-01-01), pages 92-104, XP009143641, ISSN: 0085-2538

## Description

### Technical Field

The present invention relates to an agent for use in the treatment of a glomerular disease containing a colestimide as an active ingredient, wherein the glomerular disease is diabetic nephropathy or JgA nephropathy.

### Background Art

Renal disease is roughly divided according to the portion affected into glomerular disease, tubulointerstitial disease, and renovascular disease, of which glomerular disease is recognized as being highly prevalent. Glomerular disease begins with derangement in the complicated glomerular structure due to a disturbance of any of the components of the glomerulus, i.e., basal membrane, mesangial cells, mesangial matrix, epithelial cells, and endothelial cells. Although the etiology thereof involves a broad range of factors, including immunological mechanisms, metabolic disorders, and infectious diseases, almost all cases are discovered by manifestation of proteinuria or hematuria. In acute glomerular diseases due to infectious disease and the like, many patients achieve an amelioration of the condition in several months; however, in chronic glomerular diseases such as diabetic nephropathy and IgA nephropathy, functional nephrons decrease because of glomerular disorder, and an increase in glomerular plasma flow and a rise in glomerular capillary pressure are caused by hyperglycemia, hypertension and the like. Thereby, glomerular cell disorders are caused, which in turn advance to glomerulosclerosis. Hence, the number of functional nephrons shows a further decrease due to glomerulosclerosis, which in turn leads to end-stage renal disease in a series of vicious circles.

As such, glomerular disease is a disease which is extremely difficult to treat if advanced to end-stage renal disease, which necessitates dialysis or renal transplantation and the like, and is also a disease that poses enormous monetary burdens on society. Therefore, it is necessary to detect glomerular disease as early as possible, and to prevent its progression after onset to the maximum possible extent.

Urinary protein is the most basic test parameter in diagnosing glomerular disease, and determining the severity thereof. In particular, in glomerular disease, the proteins found in the urine are mainly albumin, which appears in the urine earlier than other proteins. Because a number of methods of measuring urinary albumin have been established, including highly-sensitive immunonephelometry and latex agglutination, the urinary albumin level is useful as an indicator of the onset of glomerular disease and an indicator for determination of therapeutic effects.

In recent years, the fact that proteinuria produces tubular/interstitial lesions to cause nephron devastation and serve as a factor of progression of renopathies has attracting attention; suppressing proteinuria is also important in suppressing the progression of glomerular disease.

Treatment of glomerular disease in early stages requires blood pressure control, reducing protein intake, and reducing salt intake; the progression of nephropathy is suppressed by dietary therapy, exercise therapy, and drug therapy. Of these therapies, drug therapy employs angiotensin converting enzyme inhibitors and angiotensin receptor blockers. However, these are usable only when the subject patient is hypertensive; there has been a demand for a drug that is widely applicable to patients with normal blood pressure.

Meanwhile, regarding anion exchange resins known as cholesterol-reducing agents, typified by colestimide, there are a report on blood glucose level falls after administration for a given period (nonpatent reference 1) and a report on effects on diurnal changes in blood glucose level in patients with hypercholesterolemia complicated by type 2 diabetes mellitus (patent reference 1). Furthermore, as a mechanism of the action thereof, colestimide is known to possess insulin resistance ameliorating action (patent reference 2). The efficacy of colestimide in the treatment of diabetes mellitus has been suggested in some cases that have been reported to date. However, none of these reports state that a drug wherein such an anion exchange resin is an active ingredient thereof is effective in the treatment of diabetic nephropathy itself. A report is also available that some of the existing therapeutic drugs for diabetes mellitus do not possess ameliorating action on albuminuria or proteinuria, which are indexes of diabetic nephropathy. For example, regarding glibenclamide and troglitazone, there are some reports that apparent albuminuria is not ameliorated (nonpatent reference 2); drugs that are effective as therapeutic drugs for diabetes mellitus are not always effective as therapeutic drugs for diabetic nephropathy. There are also some cases where nephrotic syndrome can occur even when the blood glucose level and blood pressure control are good.

Of pharmaceutically acceptable anion exchange resins, colestimide and sevelamer hydrochloride are also known to be effective as therapeutic agents for hyperphosphatemia (patent reference 3). Although patent reference 3 states that a drug wherein such an anion exchange resin is an active ingredient thereof is effective on hyperphosphatemia that accompanies renal dysfunction, the target of treatment here is always renal dysfunction that accompanies an elevation of serum phosphorus concentration. Specifically, the index used in the treatment of hyperphosphatemia is serum phosphorius concentration lowering action, being totally different from ameliorating action on albuminuria or proteinuria, which are indexes in the treatment for glomerular disease of the present invention. Therefore, a drug that is effective on hyperphosphatemia that accompanies renal dysfunction is not always effective on glomerular disease.

WO 2005/032563 A discloses a method for treating vascular inflammation comprising the step of administering a therapeutically effective amount of an amine polymer to a subject.

Nagano Nobuo et al: "Sevelamer hydrochloride, a phosphate binder, protects against deterioration of renal function in rats with progressive chronic renal insufficiency", Nephrology Dialysis Transplantation, Vol. 18, No. 10, October 2003, Pages 2014-2023, XP002554968 discloses the protection of sevelamer hydrochloride against deterioration of renal function in rat with progressive chronic renal insufficiency.

Diamond JR et al: "Cholestyramine resin ameliorates chronic aminonucleoside nephrosis", American Journal of Clinical Nutrition, Vol. 51, No. 4, 1990, Pages 606-611, XP002554969 discloses that cholestyramine resin ameliorates chronic aminonucleoside nephrosis.

McDonald EL et al: "Oedema Due to Subacute Nephritis Treated with Ion-exchange Resins", Proceedings of the Royal Society of Medicine, Academic Press, London, GB, Vol. 46, No. 1, 1 January 1953, Pages 46-48, XP009125636 discloses the incidence of oedema due to subacute nephritis treated with ion-exchange resins.

Rosenheim ML et al: "Treatment of nephrotic syndrome with cation-exchange resins and high-protein low-sodium diet", Lancet The, Lancet Limited, London, GB, Vol. 271, No. 6938, 18 August 1956, Pages 313-319, XP009125657 discloses the treatement of nephrotic syndrome with cation-exchange resins and high-protein low-sodium diet.

EP 1 413 309 A discloses drugs for ameliorating postcibal hyperglycemia comprising pharmaceutically acceptable anion exchange resins such as colestimide.
patent reference 1: WO03/011398
patent reference 2: WO05/092349
patent reference 3: EP-A-793960
nonpatent reference 1: Rinsho Iyaku, vol. 12, No. 8, June 1996, p. 1641
nonpatent reference 2: Diabet Med. 2001 Apr.; 18(4): 308-13

### Disclosure of the Invention

### Problem to Be Solved by the Invention

It is an object of the present invention to provide a novel prophylactic and/or therapeutic agent for a glomerular disease, wherein a pharmaceutically acceptable anion exchange resin is an active ingredient thereof.

### Means of Solving the Problem

The present inventors diligently investigated with the aim of solving the above-described problem, found that colestimide, which is known as a pharmaceutically acceptable anion exchange resin, and the like, cause reductions in urinary albumin level, and developed the present invention.

Accordingly, the scope of the present invention is as desccribed in claims 1 - 8.

### Effect of the Invention

According to the present invention, it is possible to provide a novel drug that is effective in the prophylaxis and/or treatment of glomerular disease. In particular, according to the present invention, it is possible to provide a drug that can be used in combination with drugs currently in use for the treatment of glomerular disease, such as angiotensin converting enzyme inhibitors and angiotensin receptor blockers, and that is applicable to patients with normal blood pressure.

### Brief Description of the Drawings

FIG. 1 is a graph showing changes in urinary albumin and HbA1c in case 1 in Example 1.
FIG. 2 is a graph showing changes in urinary albumin and HbA1c in case 2 in Example 1.
FIG. 3 is a graph showing changes in urinary albumin and HbA1c in case 3 in Example 1.
FIG. 4 is a graph showing changes in urinary albumin and HbA1c in case 4 in Example 1.
FIG. 5 is a graph showing changes in urinary albumin and HbA1c in case 5 in Example 1.
FIG. 6 is a graph showing changes in urinary albumin and HbA1c in case 6 in Example 1.
FIG. 7 is a graph showing changes in urinary albumin and HbA1c in case 7 in Example 1.
FIG. 8 is a graph showing changes in urinary albumin and HbA1c in case 8 in Example 1.
FIG. 9 is a graph showing changes in urinary albumin and HbA1c in case 9 in Example 1.
FIG. 10 is a graph showing changes in urinary albumin and HbA1c in case 1 in Example 2.
FIG. 11 is a graph showing changes in urinary albumin and HbA1c in case 2 in Example 2.
FIG. 12 is a graph showing changes in urinary albumin and HbA1c in Example 3.

### Best Mode for Carrying out the Invention

The present invention is hereinafter described in greater detail.

In the present invention, a pharmaceutically acceptable anion exchange resin is an anion exchange resin that can be administered as a pharmaceutical, and is preferably an anion exchange resin having the capability of adsorbing bile acid.

The resin used in the present invention is colestimide (2-methylimidazole-epichlorohydrin copolymer). Colestimide has an irregularly deranged complex steric structure, shown by the basic structure of the formula (I) below, which structure is partially shown by the formula (II) below, and is obtained by a polymerization reaction of an amine typified by an epichlorohydrin derivative and an imidazole derivative, by the method of production described in JP-A-SHO-60-209523.

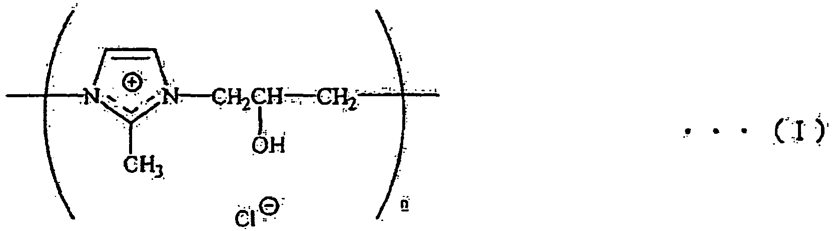

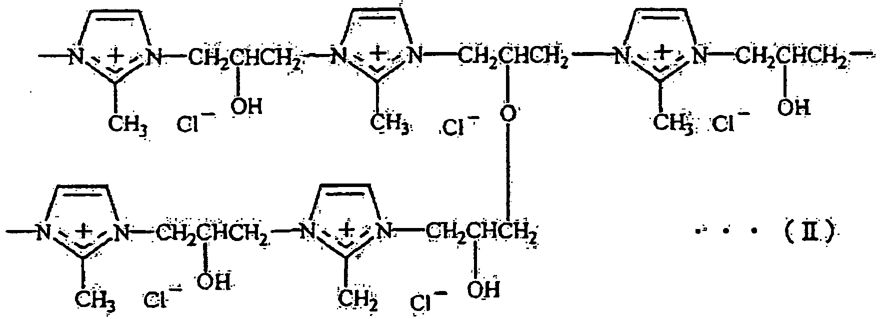

Colestimide is registered with JAN under the nonproprietary name colestimide (chemical name: 2-methylimidazole-epichlorohydrin copolymer), and registered with INN under the nonproprietary name colestilan (chemical name: 2-methylimidazole polymer with 1-chloro-2,3-epoxypropane).

Although the drug of the present invention may be used in the form of the above-described compound being an active ingredient thereof, it is preferable that the drug of the present invention be used in the form of a pharmaceutical composition containing the active ingredient, produced using commonly used additives for pharmaceutical making.

Such pharmaceutical compositions include tablets, capsules, fine granules, pills, troches, liquids and the like, and these are administered orally (including sublingual administration).

A pharmaceutical composition for oral administration can be produced by a conventional method in common use such as mixing, filling or tabletting. An active ingredient may be distributed in a pharmaceutical composition incorporating a large amount of filler, using repeated blending operation. For example, tablets or capsules for oral administration are preferably supplied as unit dosage forms, and may contain carriers for pharmaceutical making in common use, such as binders, fillers, diluents, tableting agents, lubricants, disintegrants, coloring agents, flavoring agents and wetting agents. Tablets may be prepared as coated tablets by a method obvious to those skilled in the art, using, for example, a coating agent.

Preferred filling agents include cellulose, mannitol, lactose and the like; starch, polyvinylpyrrolidone, starch derivatives such as sodium starch glycolate and the like, which are disintegrants, sodium lauryl sulfate and the like, which are lubricants, can be used as additives for pharmaceutical making. A pharmaceutical composition in the form of a liquid for oral administration is supplied as, for example, a pharmaceutical composition such as an aqueous or oily suspension, solution, emulsion, syrup or elixir, or as a dry pharmaceutical composition that can be re-dissolved in water or an appropriate medium before use.

Such liquids can be formulated with ordinary additives, for example, anti-precipitation agents such as sorbitol, syrup, methylcellulose, gelatin, hydroxyethylcellulose, carboxymethylcellulose, aluminum stearate gel and hydrogenated food fats; emulsifiers such as lecithin, sorbitan monooleate, and gum arabic; oily esters such as almond oil, rectified coconut oil, and glycerin ester; non-aqueous media such as propylene glycol and ethyl alcohol (food oils can be included); preservatives such as p-hydroxybenzoic acid methyl ester, ethyl ester or propyl ester, or sorbic acid; and, as required, ordinary flavoring agents or coloring agents and the like.

In the case of the above-described pharmaceutical compositions for oral administration, for example, tablets, capsules, fine granules and the like, an active ingredient is contained normally at 5 to 95% by weight, preferably at 25 to 90% by weight.

Colestimide is commercially available as Cholebine (registered trademark) from Mitsubishi Pharma Corporation; in the present invention, Cholebine may be used as is.

In the present invention, glomerular disease is diabetic nephropathy or IgA nephropathy.

In the present invention, IgA nephropathy refers to a case where all the criteria (1) to (3) below are fulfilled or a case where any one of A to C below is fulfilled in renal biopsy. (1) Persistent microscopic hematuria (in urinary sediment, RBC count 5 to 6/HPF or more), (2) intermittent or persistent proteinuria, (3) a serum IgA level of 315 mg/dL or more. A. Light microscopy reveals focal segmental to diffuse global (spherical) mesangial proliferative changes, B. as determined by fluorescent antibody method or enzyme antibody method, granular deposition of IgA, diffusely mainly in mesangial region, C. electron microscopy reveals deposition of high-electron-density matter in mesangial matrix, particularly mainly in paramesangial region.

IgA nephropathy, on the basis of histologic findings in renal biopsy, and in consideration of clinical findings, is classified into a good-prognosis group, a relatively-good-prognosis group, a relatively-poor-prognosis group, and a poor-prognosis group.

A good-prognosis group refers to a group of cases judged to be very unlikely to receive dialytic therapy, wherein there are mild mesangial cell proliferation and an increase in matrix only, without glomerular sclerosis/crescent formation/union to Bowman's capsule, and wherein no remarkable changes are observed in the tubules, interstitia, and blood vessels.

A relatively-good-prognosis group refers to a group of cases judged to be unlikely to undergo dialytic therapy, wherein mild mesangial cell proliferation and an increase in matrix are observed, but glomeruli showing glomerular sclerosis/crescent formation/union to Bowman's capsule account for less than 10% of all biopsied glomeruli, and wherein no remarkable changes are observed in the tubules, interstitia and blood vessels.

A relatively-poor-prognosis group refers to a group of cases judged to possibly undergo dialytic therapy in 5 years or more and within 20 years, wherein moderate, diffuse mesangial cell proliferation and an increase in matrix are observed, and glomeruli showing glomerular sclerosis/crescent formation/union to Bowman's capsule account for 10 to 30% of all biopsy glomeruli, wherein the tubular atrophy is mild, and the cell infiltration in interstitium is also mild except around some sclerotic glomeruli, and wherein mild sclerotic changes are observed in blood vessels.

A poor-prognosis group refers to a group of cases judged to possibly undergo dialytic therapy within 5 years, wherein severe, diffuse mesangial cell proliferation and an increase in matrix are observed, and glomeruli showing glomerular sclerosis/crescent formation/union to Bowman's capsule account for not less than 30% of all biopsy glomeruli, wherein the sclerosis rate is not less than 50% of all glomeruli when sclerotic portions are added to convert to global sclerosis, wherein glomeruli exhibiting compensatory hypertrophy are sometimes observed, wherein the tubular atrophy, interstitial cell infiltration and fibrosis are severe, and wherein thickening or degeneration is observed in some intrarenal arteriole walls.

In the present invention, diabetic nephropathy refers to a case that meets the criteria (1) to (5) below. (1) The length of suffering from diabetes mellitus is not less than 5 years, (2) there are other complications such as retinitis and neurosis, (3) a persistent increase in the amount of urinary protein (albumin) excreted is observed, and other causal diseases (glomerulonephritis, hypertensive renopathies, gouty kidney and the like) are ruled out, (4) there are no other urine abnormalities such as remarkable microscopic hematuria and macroscopic hematuria, and (5) in the initial stage, there are sometimes elevations of glomerular filtration rate (GFR) and renal hypertrophy.

Diabetic nephropathy, according to changes over time in urinary protein level and renal function, is staged into pre-nephropathy stage, early nephropathy stage, apparent nephropathy first half stage, apparent nephropathy second half stage, renal insufficiency stage, and dialytic therapy stage.

The pre-nephropathy stage refers to a state wherein the urinary albumin level and renal function are normal, and glomerular hyperfiltration is present.

The early nephropathy stage refers to a state wherein the urinary albumin level is 30 to 299 mg/gCr.

The apparent nephropathy first half stage refers to a state wherein the persistent proteinuria is 1 g/day or more and the glomerular filtration rate (GFR) is normal (60 ml/min or more).

The apparent nephropathy second half stage refers to a state wherein the persistent proteinuria is 1 g/day or more, and wherein GFR has decreased (30 to 60 ml/min).

The renal insufficiency stage refers to a state wherein the persistent proteinuria is 1 g/day or more, and wherein GFR has decreased remarkably (30 ml/min or less).

The dialytic therapy stage refers to a state subsequent to introduction of dialytic therapy.

The dosage of the drug of the present invention may be determined as appropriate according to the active ingredient used, the patient's age, health status, and body weight, the seriousness of the disease, the type and frequency of concurrent treatment/procedure, the profile of the desired effect and the like. In general, for colestimide, a daily dose for an adult, based on an active ingredient content of 0.5 to 60 g, may be administered once to several times daily. In the present invention, the above-described drug of the present invention can also be administered to patients already receiving an angiotensin converting enzyme inhibitor or an angiotensin receptor blocker.

In the present invention, angiotensin converting enzyme inhibitors include, but are not limited to, captopril, enalapril maleate, alacepril, delapril hydrochloride, cilazapril, lisinopril, benazepril hydrochloride, imidapril hydrochloride, temocapril hydrochloride, quinapril hydrochloride, trandolapril, perindopril erbumine and the like.

In the present invention, angiotensin receptor blockers include, but are not limited to, candesartan cilexetil, losartan potassium, valsartan, telmisartan and the like.

Furthermore, in the present invention, the drug of the present invention and the above-described angiotensin converting enzyme inhibitor or angiotensin receptor blocker can be used concurrently, separately, or sequentially. Specifically, the above-described pharmaceutical wherein a pharmaceutically acceptable anion exchange resin is an active ingredient thereof and an angiotensin converting enzyme inhibitor and the like, on the basis of a dosage determined with appropriate adjustments according to the patient's age, condition, sex, symptoms and the like, can be administered as a single pharmaceutical composition, respectively, and can also be administered as separate pharmaceutical compositions. When administered as separate pharmaceutical compositions, the same can also be administered in the same or different dosage forms, concurrently. Furthermore, the same can also be administered in the same or different dosage forms, at different times within the same day, or at given intervals for several days, several weeks or several months according to the patient's age, condition, sex, symptoms and the like.

For the above-described angiotensin converting enzyme inhibitor and the like, a supply commercially available as a reagent may be used, and, if already available as a pharmaceutical in the market, it may be used. In the present invention, normal blood pressure refers to a state wherein the systolic pressure is not more than 130 mmHg and the diastolic pressure is not more than 80 mmHg.

Because the drug of the present invention, as described in an Example below, lowers the urinary albumin level in patients suffering from glomerular disease, it is useful as a prophylactic and/or therapeutic agent for glomerular disease, and effective on a broad range of degrees of progression of nephropathy, from the pre-nephropathy stage to the renal insufficiency stage in diabetic nephropathy, and from a good-prognosis group to a poor-prognosis group in IgA nephropathy. Unlike angiotensin converting enzyme inhibitors and the like that have conventionally been used to treat glomerular disease, the drug of the present invention can also be used for patients with normal blood pressure.

### Examples

The present invention is hereinafter described specifically by reference to the following examples, to which, however, the present invention is not limited. The supply of colestimide used below was Cholebine (registered trademark) Mini 83%, commercially available from Mitsubishi Pharma Corporation, unless otherwise stated.

### Example 1

### (Subjects and methods)

Colestimide was administered to outpatients (adult, either sex) with hypercholesterolemia complicated by type 2 diabetes mellitus who exhibited microalbuminuria (30 mg/g.Cr or more and less than 300 mg/g.Cr) or apparent albuminuria (300 mg/g.Cr or more) in casual urine, and serum lipid, HbA1c (%) and casual urinary albumin levels were monitored at intervals of about 3 months for about 6 months or more.

The study schedule was as shown below.

### (1) Observation period:

It was confirmed that the serum lipid level and HbA1c were stable in the observation period, and that there was no change in the concomitant medications for 1 year or more.

### (2) Treatment period:

At intervals of about 3 months after the start of administration of colestimide, serum lipid, HbA1c and urinary albumin levels were compared with those obtained in the observation period for 6 months or more.

### (Test drugs and dosage and administration)

Cholebine Mini 83% (commercially available from Mitsubishi Pharma Corporation), in a dose of 1.5 g based on colestimide, was taken twice a day, before breakfast and dinner. In case 5, however, Cholebine Tablets 500 mg (commercially available from Mitsubishi Pharma Corporation), in a dose of 0.5 g based on colestimide, was taken once to twice a day, before breakfast and dinner.

### (Dietary therapy and concomitant medications)

Throughout the observation period and the treatment period, a constant calorific value was specified (about 30 kcal per kg desired body weight). Throughout the observation period and the treatment period, if any drug that can influence the serum lipid level (HMG-CoA reductase inhibitors, fibrate-series drugs) had been administered, a change in the dosage and administration was prohibited throughout the observation period and the treatment period, and supplementary administration was prohibited. If any drug that can influence the blood glucose level (sulfonylurea drugs, α-glucosidase inhibitors, insulin) had been administered, a change in the dosage and administration was prohibited throughout the observation period and the treatment period, and supplementary administration was prohibited. If any drug that can influence the urinary albumin level (angiotensin converting enzyme inhibitors, angiotensin receptor blockers, calcium channel antagonists) had been administered, the administration thereof was continued, without changing the dosage and administration, throughout the observation period and the treatment period, and supplementary administration of these drugs was prohibited. For other drugs, supplementary administration, a change in the dosage and administration, and discontinuation of administration in the treatment period were permitted.

### (Results)

The subject patients and the results therefrom are shown below. As shown below, TC indicates total cholesterol levels (mg/dL), HbA1c indicates glycohemoglobin A1c levels (%), Cr indicates creatinine levels (mg/dL), and the numerical values preceding or following each arrow indicate levels obtained in the observation period before colestimide administration and every 3 months.

In all figures, ● indicates urinary albumin levels, ■ indicates HbA1c levels, the axis of abscissas indicates measuring points at 3-month intervals, the left axis of ordinates indicates urinary albumin levels (mg/g.Cr), and the right axis of ordinates indicates HbA1c (%).

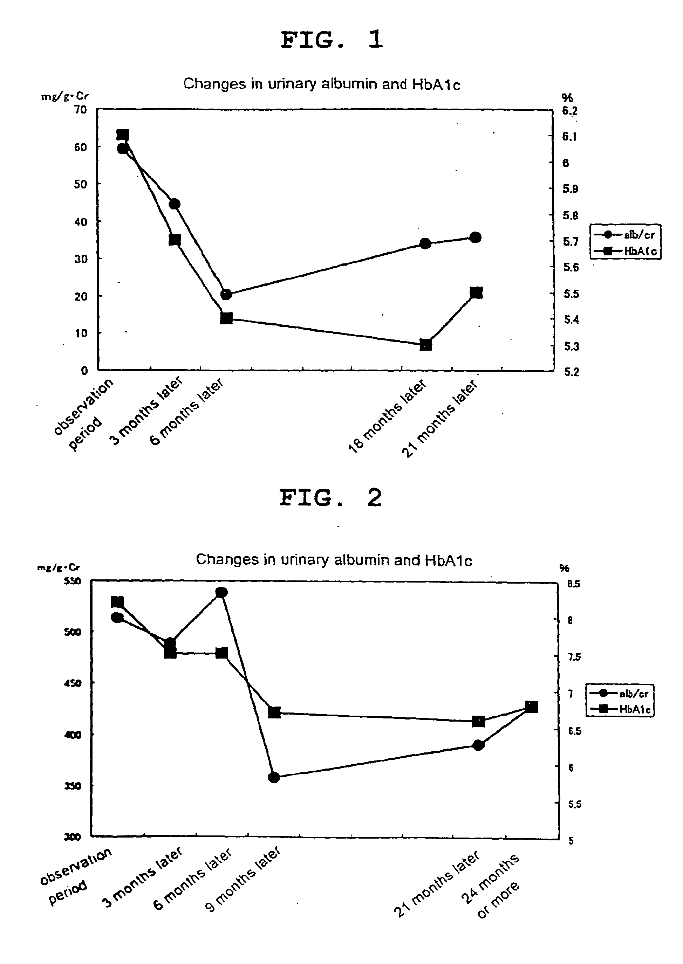

Case 1: A 66-year-old male, aspirin (nonproprietary name: administered at 100 mg/day), isosorbide mononitrate (nonproprietary name: administered at 40 mg/day), sennoside (nonproprietary name: administered at 24 mg/day)
TC 166→169→147→148→140→155→141→160
Cr 0.9→0.9→0.9→0.8→0.8→0.8→0.9→0.9
Changes in casual urinary albumin and HbA1c are shown in FIG. 1.

Case 2: A 75-year-old male, isosorbide dinitrate sustained-release tablets (nonproprietary name: administered at 60 mg/day), dilazep dihydrochloride (nonproprietary name: administered at 300 mg/day), acarbose (nonproprietary name: administered at 300 mg/day), theophylline sustained-release preparation (nonproprietary name: administered at 200 mg/day), glibenclamide (nonproprietary name: administered at 2.5 mg/day), tulobuterol patch (nonproprietary name: administered at 2 mg/day), benidipine hydrochloride (nonproprietary name: administered at 4 mg/day), rebamipide (nonproprietary name: administered at 300 mg/day)
TC 208→219→223→205→218→222→189→220→213
Cr 0.6→0.6→0.6→0.6→0.6→0.6→0.6→0.6→0.6
Changes in casual urinary albumin and HbA1c are shown in FIG. 2.

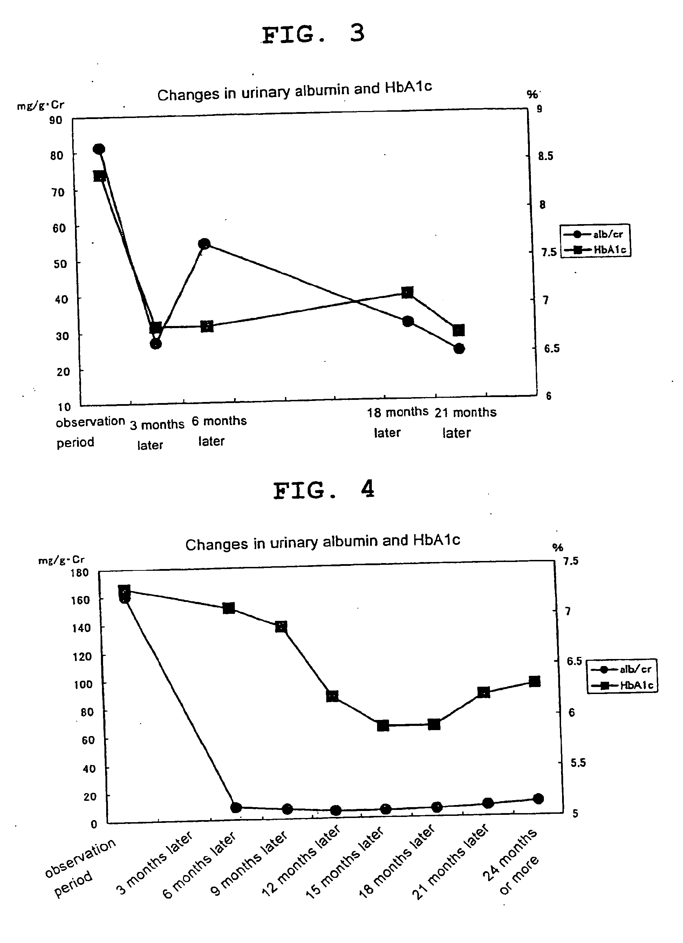

Case 3: An 85-year-old female, fenofibrate (nonproprietary name: administered at 100 mg/day), barnidipine hydrochloride (nonproprietary name: administered at 10 mg/day), levothyroxine sodium (T₄) (nonproprietary name: administered at 50 µg/day), aspirin (nonproprietary name: administered at 81 mg/day), candesartan cilexetil (nonproprietary name: administered at 4 mg/day), etizolam (nonproprietary name: administered at 0.5 mg/day)
TC 211→165→179→179→169→168→164
Cr 0.5→0.7→0.5→0.5→0.6→0.5→0.5
Changes in casual urinary albumin and HbA1c are shown in FIG. 3.

Case 4: A 57-year-old female, voglibose (nonproprietary name: administered at 0.6 mg/day), glimepiride (nonproprietary name: administered at 3 mg/day), atorvastatin calcium hydrate (nonproprietary name: administered at 10 mg/day), candesartan cilexetil (nonproprietary name: administered at 4 mg/day)
TC 197→ (not measured) →208→147→133→149→ (not measured)→146→185
Cr 0.6→ (not measured) →0.6→0.6→0. 6→0.6→ (not measured)→0.6→0.6
Changes in casual urinary albumin and HbA1c are shown in FIG. 4.

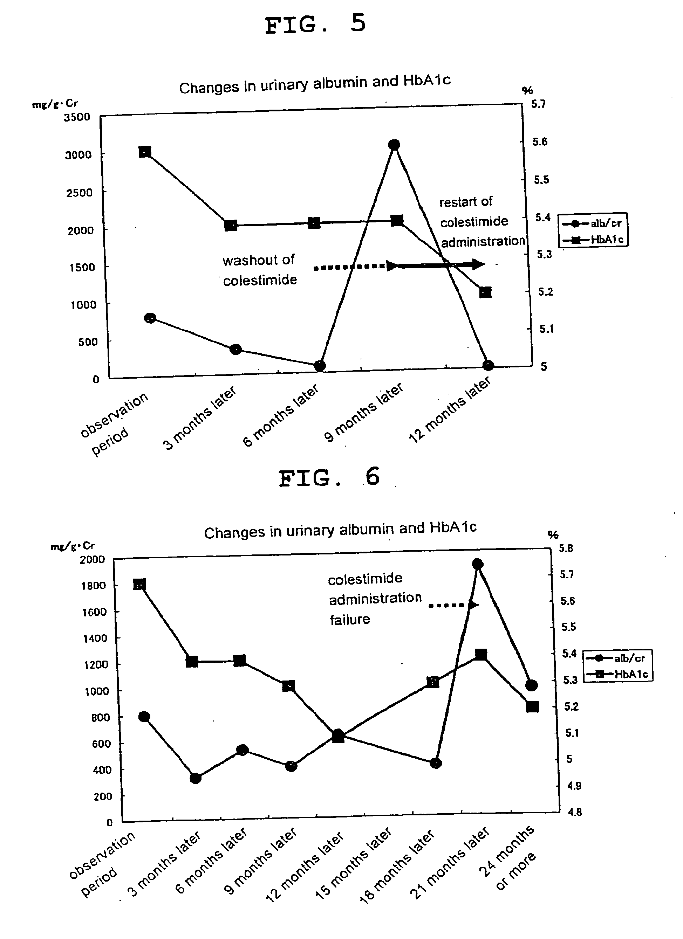

Case 5: An 81-year-old female, candesartan cilexetil (nonproprietary name: administered at 8 mg/day), amlodipine besilate (nonproprietary name: administered at 5 mg/day), allopurinol (nonproprietary name: administered at 100 mg/day), azulene sulfonate sodium (nonproprietary name: administered at 4.5 mg/day), L-glutamine (nonproprietary name: administered at 1485 mg/day)
TC (not measured) →208→220→235→ (not measured)
Cr (not measured) →0.56→0.6→0.53→ (not measured)
Changes in casual urinary albumin and HbA1c are shown in FIG. 5.

Case 6: A 65-year-old male, amlodipine besilate (nonproprietary name: administered at 5 mg/day), enalapril maleate (nonproprietary name: administered at 5 mg/day), telmisartan (nonproprietary name: administered at 40 mg/day), allopurinol (nonproprietary name: administered at 200 mg/day), sennoside (nonproprietary name: administered at 24 mg/day) TC 196→187→204→204→237→ (not measured) →196→216→204 Cr 2.0→1.8→1.7→1.8→1.7→ (not measured) →1.7→1.8→2.0 Changes in casual urinary albumin and HbA1c are shown in FIG. 6.

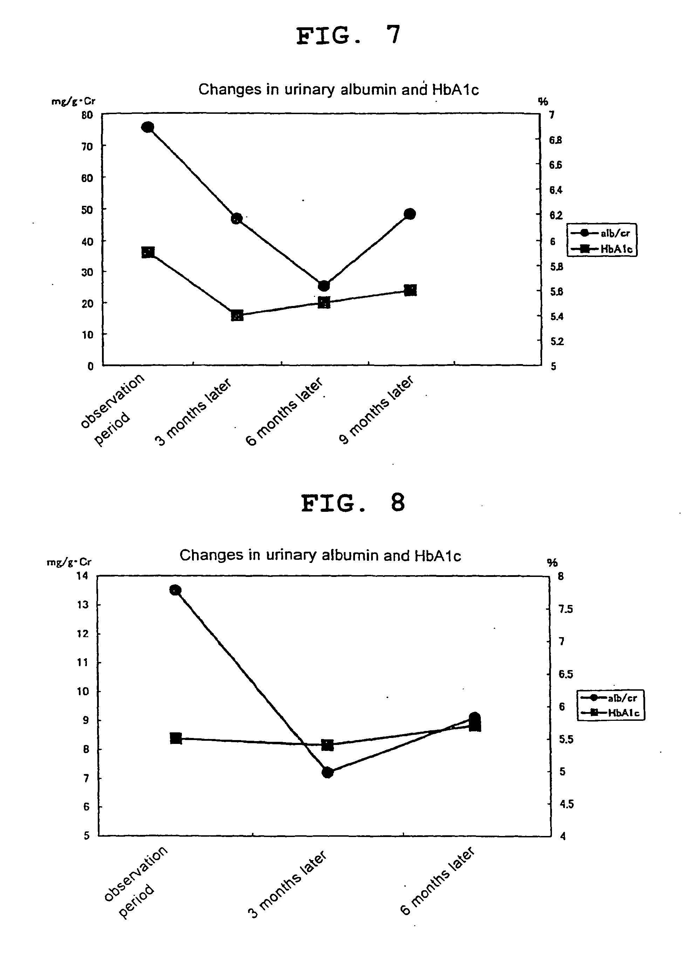

Case 7: A 74-year-old female, no concomitant medications
TC 210→178→187→177
Cr 0.5→0.5→0.5→0.6
Changes in casual urinary albumin and HbA1c are shown in FIG. 7.

Case 8: A 57-year-old female, no concomitant medications
TC 250→194→220
Cr 0.4→0.4→0.4
Changes in casual urinary albumin and HbA1c are shown in FIG. 8.

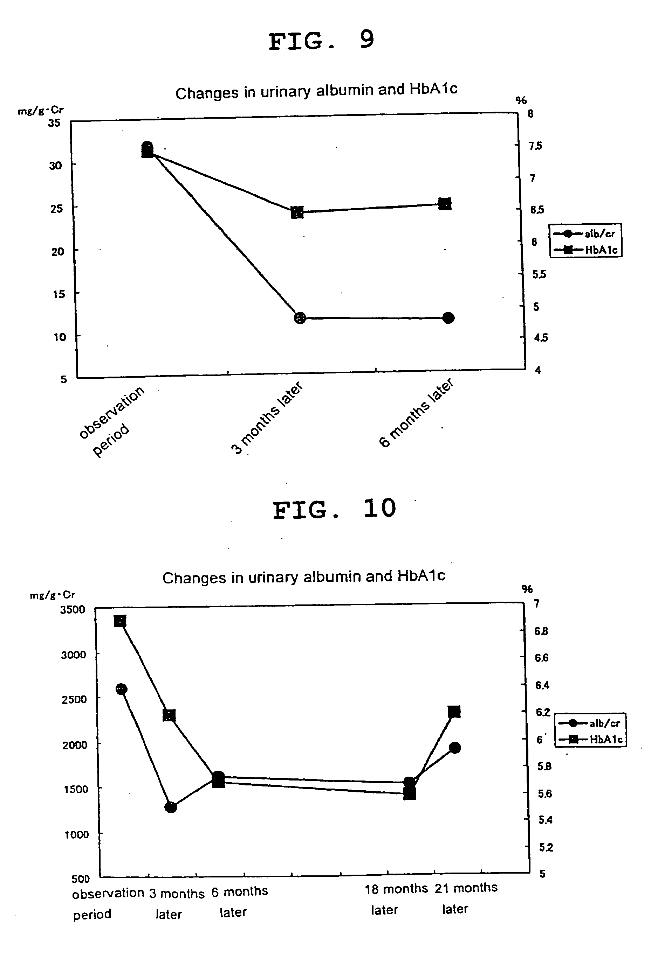

Case 9: An 86-year-old female, benidipine hydrochloride (nonproprietary name: administered at 4 mg/day), misoprostol (nonproprietary name: administered at 400 µg/day), nicorandil (nonproprietary name: administered at 10 mg/day), sennoside (nonproprietary name: administered at 12 mg/day), voglibose (nonproprietary name: administered at 0.6 mg/day), sodium risedronate hydrate (nonproprietary name: administered at 2.5 mg/day), brotizolam (nonproprietary name: administered at 0.125 mg/day)
TC 233→199→ (not measured) →186
Cr 0.4-0.4→ (not measured) →0.4
Changes in casual urinary albumin and HbA1c are shown in FIG. 9.

In all the cases described above, the casual urinary albumin level and HbA1c level decreased evidently; of the above-described results, in case 3, case 4, case 5 and case 6, angiotensin receptor blockers (which have renal protecting action and reduce urinary albumin) had been administered, and administration of colestimide further reduced the urinary albumin level. In case 6, an angiotensin converting enzyme inhibitor (which, like angiotensin receptor blockers, has renal protecting action and reduces urinary albumin) was coadministered, and even under the conditions, colestimide produced a reduction in the urinary albumin level. In case 5, when administration of colestimide only was discontinued at 6 months of administration, the HbA1c level did not change but an abrupt rise in the casual urinary albumin level was observed; on the day, colestimide was restarted at 0.5 g/day, and 2 weeks later, the dose was increased to 1.0 g/day. Three months after the restarting day, the casual urinary albumin level decreased dramatically, and a reduction in the HbA1c level was also observed. In case 6, rises in the casual urinary albumin level and HbA1c level were observed after 21 months of administration. By confirmation with the patient, a failure to take colestimide was revealed. Later, colestimide was taken in compliance with the dosage and administration schedule, and reductions in the casual urinary albumin level and HbA1c level were observed.

From this finding, it is evident that colestimide has an excellent effect on diabetic nephropathy.

### Example 2

### (Subjects and methods)

Colestimide was administered to outpatients (adult, either sex) with hypercholesterolemia complicated by type 2 diabetes mellitus who exhibited apparent albuminuria (300 mg/g .Cr or more) in casual urine, and serum lipid, HbA1c (%) and casual urinary albumin levels were monitored at intervals of about 3 months for 12 to 21 months.

The study schedule was as shown below.

### (1) Observation period:

It was confirmed that the serum lipid level and HbA1c were stable in the observation period, and that there was no change in the concomitant medications for 1 year or more.

### (2) Treatment period:

At intervals of about 3 months after the start of administration of colestimide, serum lipid, HbA1c and urinary albumin levels were compared with those obtained in the observation period for 12 to 21 months.

### (Test drug and dosage and administration)

Cholebine Mini 83% (commercially available from Mitsubishi Pharma Corporation), in a dose of 1.5 g based on colestimide, was taken twice a day, before breakfast and dinner.

### (Dietary therapy and concomitant medications)

Throughout the observation period and the treatment period, a constant calorific value was specified (about 30 kcal per kg of desired body weight). Throughout the observation period and the treatment period, for any drug that can influence the serum lipid level (HMG-CoA reductase inhibitors, fibrate-series drugs), a change in the dosage and administration was prohibited throughout the observation period and the treatment period, and supplementary administration was prohibited. For any drug that can influence the blood glucose level (sulfonylurea drugs, α-glucosidase inhibitors, insulin), adjustments of the dosage according to symptoms was permitted. For any drug that can influence the urinary albumin level (angiotensin converting enzyme inhibitors, angiotensin receptor blockers, calcium channel antagonists), the administration was continued, without changing the dosage and administration, throughout the observation period and the treatment period, and supplementary administration of these drugs was prohibited. For other drugs, a change in the dosage and administration was prohibited throughout the observation period and the treatment period.

### (Results)

The concomitant medications and the results are shown below. As shown below, TC indicates total cholesterol levels (mg/dL), HbA1c indicates glycohemoglobin A1c levels (%), Cr indicates creatinine levels (mg/dL), and the numerical figures preceding or following each arrow indicate levels obtained in the observation period before colestimide administration and every 3 months.

In all figures, ● indicates urinary albumin levels, ■ indicates HbA1c levels, the axis of abscissas indicates measuring points at 3-month intervals, the left axis of ordinates indicates urinary albumin levels (mg/g.Cr), and the right axis of ordinates indicates HbA1c (%).

Case 1: A 62-year-old male, aspirin (nonproprietary name: administered at 100 mg/day), valsartan (nonproprietary name: administered at 40 mg/day), voglibose (nonproprietary name: administered at 0.9 mg/day), isophane insulin aqueous suspension (nonproprietary name: administered at 8 units/day (for 10 months) → units/day (for 8 months) →4 units/day)
TC 205→164→159→ (not measured) →181→166→164→183
Cr 1.2→1.3→1.3→ (not measured) →1.4→1.3→1.4→1.4
Changes in casual urinary albumin and HbA1c are shown in FIG. 10.

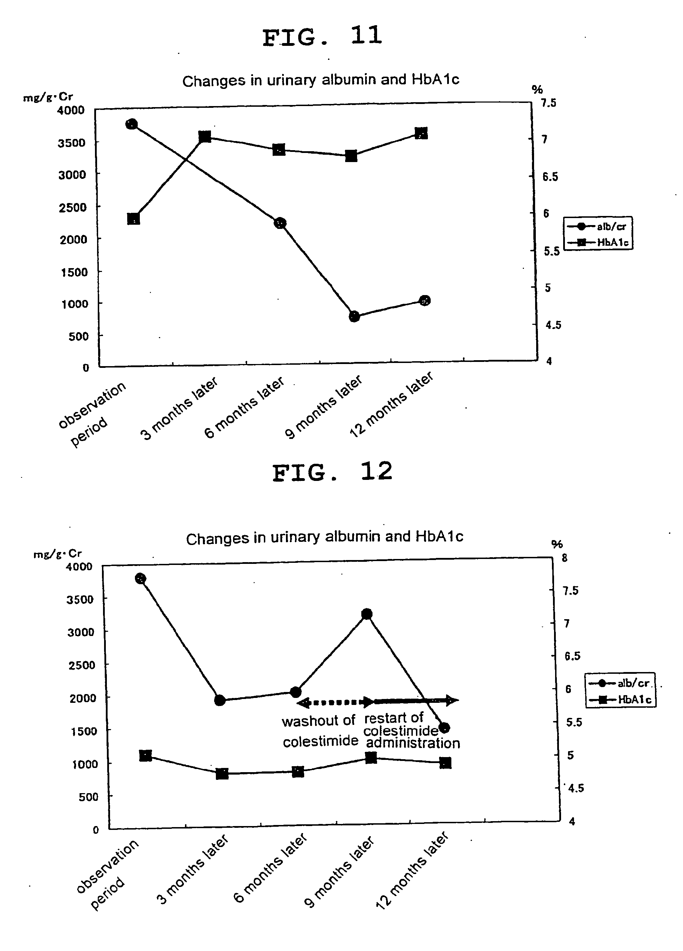

Case 2: An 81-year-old female, biosynthesized human biphasic isophane insulin aqueous suspension (nonproprietary name: administered at 10 units/day (for 7 months)→6 units/day (for 3.5 months)→8 units/day), amlodipine besilate (nonproprietary name: administered at 2.5 mg/day), aspirin (nonproprietary name: administered at 100 mg/day), sodium ferrous citrate (nonproprietary name: administered at 100 mg/day), cilostazol (nonproprietary name: administered at 100 mg/day), pravastatin sodium (nonproprietary name: administered at 10 mg/day)
TC 236→ (not measured)→199→224→204
Cr 0.5→ (not measured) →0.5→0.6→0.6
Changes in casual urinary albumin and HbA1c are shown in FIG. 11.

In all the cases described above, casual urinary albumin decreased evidently; of the above-described results, in case 1, an angiotensin receptor blocker (which has renal protecting action and reduces urinary albumin) had been administered, and administration of colestimide further reduced the casual urinary albumin level. In case 2, a calcium channel antagonist (which has renal protecting action and reduces urinary albumin) had been administered, and administration of colestimide further reduced the casual urinary albumin level.

From this finding, it is evident that colestimide has an excellent effect on diabetic nephropathy, even when used in combination with a drug having renal protecting action.

In case 2, the HbA1c level rose in the treatment period compared to the observation period, but the casual urinary albumin level decreased evidently in the treatment period.

From this finding, it is evident that the excellent effect of colestimide on diabetic nephropathy is not associated with the conventionally known hypoglycemic action of colestimide at all.

From this finding, it is evident that colestimide has an excellent effect on diabetic nephropathy, and that this effect was not easily arrived at by those skilled in the art.

### Example 3

### (Subject and methods)

Colestimide was administered to an outpatient (72-year-old male) with hypercholesterolemia complicated by IgA nephropathy (serum IgA level 414 mg/dl) who exhibited apparent albuminuria (300 mg/g.Cr or more) in casual urine for 6 months, and this was followed by a washout of 3 months and subsequent re-administration for 3 months. During that period, serum lipid, HbA1c (%) and casual urinary albumin levels were monitored at intervals of about 3 months.

The study schedule was as shown below.

### (1) Observation period:

It was confirmed that the serum lipid level was stable and HbA1c was in the normal range in the observation period, and that there was no change in the concomitant medications for 1 year or more.

### (2) Treatment period:

Colestimide was administered for 6 months, and this was followed by a washout of 3 months and subsequent re-administration for 3 months; the serum lipid, HbA1c and urinary albumin levels obtained at intervals of about 3 months for 12 months were compared with those obtained in the observation period and the washout period.

### (Test drug and dosage and administration)

Cholebine Mini 83% (commercially available from Mitsubishi Pharma Corporation), in a dose of 1.5 g based on colestimide, was taken twice a day, before breakfast and dinner.

### (Dietary therapy and concomitant medications

Throughout the observation period and the treatment period, reducing salt intake (less than 6 g/day) and reducing protein intake (less than 0.8 g/kg/day) were implemented. Throughout the observation period and the treatment period, supplementary administration of any drug that can influence the serum lipid level (HMG-CoA reductase inhibitors, fibrate-series drugs) was prohibited throughout the observation period and the treatment period. For any drug that can influence the urinary albumin level (angiotensin converting enzyme inhibitors, angiotensin receptor blockers, calcium channel antagonists), administration was continued, without changing the dosage and administration, throughout the observation period and the treatment period, and supplementary administration of these drugs was prohibited. For other drugs, a change in the dosage and administration was prohibited throughout the observation period and the treatment period.

### (Results)

The concomitant medications and the results obtained therewith are shown below. As shown below, TC indicates total cholesterol levels (mg/dL), HbA1c indicates glycohemoglobin A1c levels (%), Cr indicates creatinine levels (mg/dL), and the numerical values preceding or following each arrow indicate levels obtained in the observation period before colestimide administration and every 3 months.

In the figure, ● indicates urinary albumin levels, ■ indicates HbA1c levels, the axis of abscissas indicates measuring points at 3-month intervals, the left axis of ordinates indicates urinary albumin levels (mg/g.Cr), and the right axis of ordinates indicates HbA1c (%).

Concomitant medications: allopurinol (nonproprietary name: administered at 200 mg/day), imidapril hydrochloride (nonproprietary name: administered at 10 mg/day), amlodipine besilate (nonproprietary name: administered at 5 mg/day), aspirin (nonproprietary name: administered at 100 mg/day)
TC 220→182→136→200→172
Cr 1.4→1.4→1.3→1.2→1.4
Changes in casual urinary albumin and HbA1c are shown in FIG. 12.

In the above-described case, calcium channel antagonists (which have renal protecting action and lower the urinary albumin level) had been administered, and administration of colestimide further lowered the urinary albumin level. The casual urinary albumin level decreased in the colestimide administration period, but increased in the washout period, and decreased again with re-administration of colestimide.

From this finding, it is evident that colestimide has an excellent effect on IgA nephropathy.

### Industrial Applicability

According to the present invention, a drug that ameliorates glomerular disease is obtained. This action is also observed when the drug is used in combination with an angiotensin converting enzyme inhibitor or angiotensin receptor blocker whose efficacy for glomerular diseases such as diabetic nephropathy and IgA nephropathy has been established. Without hypotensive action, the drug can also be used for patients with normal blood pressure.

## Claims

1. An agent for use in the treatment of a glomerular disease, comprising colestimide as an active ingredient, wherein the glomerular disease is diabetic nephropathy or IgA nephropathy.

2. The agent according to claim 1 for use according to claim 1, wherein the glomerular disease is diabetic nephropathy.

3. The agent according to claim 1 for use according to claim 1 or 2, wherein the diabetic nephropathy is in the early nephropathy stage.

4. The agent according to claim 1 for use according to claim 1, wherein the glomerular disease is IgA nephropathy.

5. The agent according to claim 1 for use according to any one of claims 1 to 4, which reduces the urinary albumin level.

6. The agent according to claim 1 for use according to any one of claims 1 to 5, to be used in patients already receiving an angiotensin converting enzyme inhibitor or an angiotensin receptor blocker.

7. The agent according to claim 1 for use according to any one of claims 1 to 6, wherein an angiotensin converting enzyme inhibitor or an angiotensin receptor blocker is used concurrently, separately, or sequentially.

8. The agent according to claim 1 for use according to any one of claims 1 to 7, to be used for patients with normal blood pressure.

## Patentansprüche

1. Mittel zur Verwendung in der Behandlung einer Glomerularerkrankung, umfassend Colestimid als Wirkstoff, wobei die Glomerularerkrankung diabetische Nephropathie oder IgA-Nephropathie ist.

2. Mittel gemäß Anspruch 1 zur Verwendung gemäß Anspruch 1, wobei die Glomerularerkrankung diabetische Nephropathie ist.

3. Mittel gemäß Anspruch 1 zur Verwendung gemäß Anspruch 1 oder 2, wobei die diabetische Nephropathie Nephropathie im Frühstadium ist.

4. Mittel gemäß Anspruch 1 zur Verwendung gemäß Anspruch 1, wobei die Glomerularerkrankung IgA-Nephropathie ist.

5. Mittel gemäß Anspruch 1 zur Verwendung gemäß irgendeinem der Ansprüche 1 bis 4, welches das Harnalbuminlevel verringert.

6. Mittel gemäß Anspruch 1 zur Verwendung gemäß irgendeinem der Ansprüche 1 bis 5, zur Verwendung in Patienten, welche bereits einen Angiotensin Converting Enzyme-Inhibitor oder einen Angiotensin-Rezeptorblocker erhalten.

7. Mittel gemäß Anspruch 1 zur Verwendung gemäß irgendeinem der Ansprüche 1 bis 6, wobei ein Angiotensin Converting Enzyme-Inhibitor oder ein Angiotensin-Rezeptorblocker simultan, getrennt voneinander oder sequentiell verwendet wird.

8. Mittel gemäß Anspruch 1 zur Verwendung gemäß irgendeinem der Ansprüche 1 bis 7, zur Verwendung bei Patienten mit normalen Blutdruck.

## Revendications

1. Agent pour une utilisation dans le traitement d'une maladie glomérulaire, comprenant du colestimide en tant que principe actif, où la maladie glomérulaire est une néphropathie diabétique ou une néphropathie à IgA.

2. Agent selon la revendication 1 pour une utilisation selon la revendication 1, où la maladie glomérulaire est une néphropathie diabétique.

3. Agent selon la revendication 1 pour une utilisation selon la revendication 1 ou 2, où la néphropathie diabétique est au stade précoce de la néphropathie.

4. Agent selon la revendication 1 pour une utilisation selon la revendication 1, où la maladie glomérulaire est une néphropathie à IgA.

5. Agent selon la revendication 1 pour une utilisation selon l'une quelconque des revendications 1 à 4, qui réduit le taux d'albumine urinaire.

6. Agent selon la revendication 1 pour une utilisation selon l'une quelconque des revendications 1 à 5, à utiliser chez des patients recevant déjà un inhibiteur de l'enzyme de conversion de l'angiotensine ou un agent bloquant le récepteur de l'angiotensine.

7. Agent selon la revendication 1 pour une utilisation selon l'une quelconque des revendications 1 à 6, où un inhibiteur de l'enzyme de conversion de l'angiotensine ou un agent bloquant le récepteur de l'angiotensine est utilisé simultanément, séparément ou séquentiellement.

8. Agent selon la revendication 1 pour une utilisation selon l'une quelconque des revendications 1 à 7, à utiliser pour des patients ayant une pression sanguine normale.
